# EUROPEAN PATENT SPECIFICATION

(11) **EP 3 407 858 B1**
(45) Date of publication and mention of the grant of the patent: **13.10.2021**
(21) Application number: 17744674.7
(22) Date of filing: 11.01.2017
(51) Int. Cl.: A61K 8/35, A61K 8/97, A61Q 19/00, A61Q 5/00, A61Q 5/02, A61Q 5/12, A61K 8/9789, A61K 8/9783, A61Q 7/00, A61K 31/12, A61K 36/03, A61K 36/185, A61K 8/9711

(54) **A METHOD FOR TREATING THE APPEARANCE OF THINNING HAIR**
VERFAHREN ZUR BEHANDLUNG DES AUSSEHENS VON DÜNNER WERDENDEM HAAR
MÉTHODE DE TRAITEMENT DE L'ASPECT DÉGARNI DES CHEVEUX

(30) Priority: 27.01.2016 US 201662287496 P
(43) Date of publication of application: 05.12.2018
(73) Proprietor: ELC Management LLC, Melville, NY 11747 (US)
(72) Inventor: PAWLUS, Alison, Saint Paul Minnesota 55108 (US); HAWKINS, Geoffrey, Yardley Pennsylvania 19067 (US)
(74) Representative: Dehns
(86) International application number: PCT/US2017/012981
(87) International publication number: WO 2017/131956

(56) References cited:
- EP-A1- 2 853 254
- WO-A1-2009/046116
- JP-A- H02 142 717
- JP-A- 2006 028 118
- US-A1- 2003 224 028
- US-A1- 2006 008 428
- US-A1- 2006 210 515
- US-A1- 2007 036 742
- US-B2- 7 205 009

## Description

### FIELD OF THE INVENTION

The present invention relates to use of personal care compositions containing a synergistic blend of naturally derived 5-alpha reductase inhibitors, which provide a hair restorative benefit.

### BACKGROUND OF THE INVENTION

Hair loss is a common skin disorder that affects hair follicles and is characterized by thinning, typically starting at the temples or the crown in men and parietal region in women; continued thinning without treatment leads to atrophy and total loss of hair follicles, which leads to baldness. While the condition is not life threatening and does not endanger health, it leads to social anxiety and other psychological consequences for many sufferers.

A variety of hair loss treatment methods have been developed, including topical minoxidil, finasteride and various other antiandrogens, laser therapy, corticosteroid injections, oral contraceptives, and surgical procedures such as hair transplantation. The uses of existing therapies, however, have certain disadvantages.

Oral finasteride, an effective treatment for many patients, has had a significant number of reported side effects including decreased libido, erectile dysfunction, ejaculatory dysfunction, and myopathy.

Another popular treatment is topical minoxidil. Topical minoxidil requires *in vivo* activation to its active form, minoxidil sulfate, by a sulfotransferase enzyme, whose expression is variable among individuals. Finasteride is a potent inhibitor of 5-alpha reductase, preventing the conversion of testosterone to dihydrotestosterone (DHT). Minoxidil's mechanism of action is not completely understood, however it is know to act as a potassium ion-channel opener, increases blood flow via vasodilation, and stimulates cellular proliferation *in vitro.* Currently, there is limited information available concerning topical use of finasteride, and its oral treatment is known to have a multitude of side effects. Meanwhile, minoxidil is used topically, but has a failure rate greater than 50% along with side effects such as redness, irritation, and burning. It is believed that finasteride has limited topical efficacy before cytotoxicity is observed in dermal papilla cells, and therefore its topical efficacy is limited.

The risks and costs associated with hair transplantation are well known and its usefulness is limited by the number of hair grafts that can be transplanted to the affected area. Patients who have little hair loss and have a successful transplant still require ongoing topical or oral therapy to prevent the surrounding hair (the non-transplanted hairs) from falling out. These patients are also subjected to the disadvantages of topical minoxidil and oral finasteride.

For these reasons, it would be desirable to provide improved compositions and methods for the treatment of hair loss, hair thinning, and/or alopecia. In particular, it would be desirable to provide products with effective hair restorative benefits with minimized side effects or cytotoxicity. And there is a further desire to provide a solution which is sourced from naturally occurring materials.

US 2003/0224028A discloses a cosmetic composition comprising at least one metal complex and a physiologically acceptable medium that is intended to promote desquamation of the skin and/or to stimulate epidermal renewal.

US 2006/0008428A discloses a method and composition for promoting the penetration of a cosmetic active.

### SUMMARY OF THE INVENTION

The present invention is directed to a non-therapeutic method for treating the appearance of thinning hair of a human comprising the steps of; cleaning the hair with a shampoo composition; rinsing said shampoo from said hair; and applying a hair restorative blend to the scalp of said human, said hair restorative blend comprising; a chalconoid, an Epilobium extract selected from *Epilobium fleischeri* extract or *Epilobium angustifolium* extract, and a cosmetically acceptable carrier.

These and other features, aspects, and advantages of the present invention will become evident to those skilled in the art from a reading of the present disclosure.

### DETAILED DESCRIPTION OF THE INVENTION

While the specification concludes with claims that particularly point out and distinctly claim the invention, it is believed the present invention will be better understood from the following description.

All percentages, parts and ratios are based upon the total weight of the compositions of the present invention, unless otherwise specified. All such weights as they pertain to listed ingredients are based on the active level, and therefore they do not include solvents or by-products that may be included in commercially available materials, unless otherwise specified. The term "weight percent" may be denoted as "wt%" herein.

All molecular weights as used herein are weight average molecular weights expressed as grams/mole, unless otherwise specified.

All ratios are weight ratios unless specifically stated otherwise.

Herein, "*µ*" means microns.

Herein, "cs" means centistoke.

Herein, "molecular weight" is measured in terms of the weight average molecular weight, and is measured by gel permeation chromatography (GPC).

Herein, "graft" means attached to a backbone at any position other than an end group.

The term "water-soluble," as used herein, means that the polymer is soluble in water in the present composition. In general, the polymer should be soluble at 25 °C at a concentration of at least 0.1% by weight of the water solvent, preferably at least 1%, more preferably at least 5%, most preferably at least 15%.

The term "water-insoluble," as used herein, means that a compound is not soluble in water in the present composition. Thus, the compound is not miscible with water.

### Hair Restorative Blend

The personal care compositions may include a blend of at least three hair restorative materials, which, when combined according to the present disclosure, create a hair restorative blend.

The first hair restorative material is a chalconoid. Nonlimiting examples of suitable chalconoids are selected from the group consisting of chalcone, chalconoid, butein, cardamomin, isoliquiritigenin, licochalcone A, licochalcone B, licochalcone C, licochalcone D, licochalcone E, sophoradin, xanthohumol, methyl hydroxychalcone, okanin, xanthohumol, and mixtures thereof. The chalconoid may be present in the composition at a level of from about 0.1% to about 30%, more preferably from about 1% to about 20%, and most preferably about 5% of the hair restorative blend. A particularly preferred commercial chalconoid is Licochalcone LR-15, available from Barnet®. This material is available as a powderized extract of a *Glycyrrhiza glabra* root, optimized to contain a standardized extract of approximately 20% of licochalcone A.

The second hair restorative material is an *Epilobium* extract. In the hair restorative blend used in the method of the invention the *Epilobium* extract is selected from *Epilobium fleischeri* extract or *Epilobium angustifolium* extract. Such extracts are commercially available under the trade name, Alpaflor® Alp®-Sebum, available from Centerchem, which contains from 5 to 10% of an *Epilobium fleischeri* extract. The *Epilobium* extract may be present at a level of about 30% to about 50%, preferably 40% to about 50%, and most preferably about 47.5% of the hair restorative blend. The percentages of Alpaflor Alp-Sebum provided herein are based on the 5 to 10% solution of *Epilobium fleischeri* in a solvent system.

The optional third hair restorative material is known as *Laminaria* extract. This material is commercially available under the trade name Phlorogine® CV, available from SEPPIC. The *Laminaria* extract is selected from *Laminaria saccharina* extract, *L. digitata* extract, and *L. ochroleuca* extract. The *Laminaria saccharina* extract may be present at a level of about 30% to about 60%, preferably 40% to about 50%, and most preferably about 47.5% of the hair restorative blend. The percentages of *Laminaria saccharina* provided herein are based on approximately 1.5 to 2.5% solution of *Laminaria saccharina* extract in a solvent system.

As will be discussed and exemplified herein after, the combined components of the restorative blend not only exhibit excellent hair restorative efficacy with limited cytotoxicity, but it also induces proliferation of dermal papilla cells. And as the examples illustrate, the individual components of the hair restorative blend do not exhibit dermal papilla cell proliferation enhancement with increased concentrations of individual actives. Therefore, the combined components of the hair restorative blend demonstrate an unexpected synergistic effect.

The hair restorative blends may be delivered to hair or skin in a variety of personal care product forms. For example, the hair restorative blends may be provided as shampoo compositions, conditioning compositions, leave-in conditioning compositions, serums, lotions, mousses, aerosol sprays, creams, balms, and other cosmetically or pharmaceutically acceptable product forms.

### Carrier

The hair restorative blends are provided via a cosmetically acceptable carrier, which may generally present at a level of from about 10% to about 95%, more preferably from about 60% to about 85% by weight of the composition. The carrier may be aqueous or anhydrous. Nonlimiting examples of suitable carriers include water, or a miscible mixture of water and organic solvent, ethanol or other alcohols, and mixtures thereof.

The carrier may further comprise moisture barrier enhancers to maximize efficacy of the hair restorative blends herein. Such moisture barrier enhancers include, for example, thickening agents, film-forming polymers, humectants, emoillients, at the like.

Suitable humectants are selected from, but not limited to; amino acids and derivatives thereof such as proline and arginine aspartate, 1 ,3-butylene glycol, propylene glycol and water and codium tomentosum extract, collagen amino acids or peptides, creatinine, diglycerol, biosaccharide gum-1 , glucamine salts, glucuronic acid salts, glutamic acid salts, polyethylene glycol ethers of glycerine (e.g. glycereth 20), glycerine, glycerol monopropoxylate, glycogen, hexylene glycol, honey, and extracts or derivatives thereof, hydrogenated starch hydrolysates, hydrolyzed mucopolysaccharides, inositol, keratin amino acids, urea, LAREX A-200 (available from Larex), glycosaminoglycans, methoxy PEG 10, methyl gluceth-10 and -20 (both commercially available from Amerchol located in Edison, NJ), methyl glucose, 3-methyl-1,3-butanediol, N-acetyl glucosamine salts, polyethylene glycol and derivatives thereof (such as PEG 15 butanediol, PEG 4, PEG 5 pentaerythitol, PEG 6, PEG 8, PEG 9), pentaerythitol, 1 ,2 pentanediol, PPG-1 glyceryl ether, PPG-9, 2-pyrrolidone-5-carboxylic acid and its salts such as glyceryl pea, saccharide isomerate, SEACARE (available from Secma), sericin, silk amino acids, sodium acetylhyaluronate, sodium hyaluronate, sodium poly-aspartate, sodium polyglutamate, sorbeth 20, sorbeth 6, sugar and sugar alcohols and derivatives thereof such as glucose, mannose and polyglycerol sorbitol, trehalose, triglycerol, trimethyolpropane, tris (hydroxymethyl) amino methane salts, and yeast extract, and mixtures thereof.

More preferably, the humectants for use herein are selected from glycerine, urea, butylene glycol, polyethylene glycol and derivatives thereof, and mixtures thereof. Even more preferably, the humectants for use herein are selected from glycerine, urea and mixtures thereof, especially glycerine.

### Cellulose or Guar Cationic Deposition Polymers

The personal care compositions may include additional cellulose or guar cationic deposition polymers. Generally, such cellulose or guar cationic deposition polymers may be present at a concentration of from about 0.05% to about 5%, by weight of the composition. Suitable cellulose or guar cationic deposition polymers have a molecular weight of greater than about 5,000. Additionally, such cellulose or guar deposition polymers have a charge density from about 0.5 mEq/g to about 4.0 mEq/g at the pH of intended use of the personal care composition, which pH will generally range from about pH 3 to about pH 9, preferably between about pH 4 and about pH 8. The pH of the compositions are measured neat.

Suitable cellulose or guar cationic polymers include those which conform to the following formula: 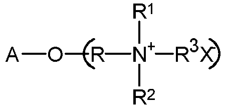 wherein A is an anhydroglucose residual group, such as a cellulose anhydroglucose residual; R is an alkylene oxyalkylene, polyoxyalkylene, or hydroxyalkylene group, or combination thereof; R¹, R², and R³ independently are alkyl, aryl, alkylaryl, arylalkyl, alkoxyalkyl, or alkoxyaryl groups, each group containing up to about 18 carbon atoms, and the total number of carbon atoms for each cationic moiety (*i.e.,* the sum of carbon atoms in R¹, R² and R³) preferably being about 20 or less; and X is an anionic counterion. Non-limiting examples of such counterions include halides (*e.g.,* chlorine, fluorine, bromine, iodine), sulfate and methylsulfate. The degree of cationic substitution in these polysaccharide polymers is typically from about 0.01 to about 1 cationic groups per anhydroglucose unit.

In one embodiment of the invention, the cellulose or guar cationic polymers are salts of hydroxyethyl cellulose reacted with trimethyl ammonium substituted epoxide, referred to in the industry (CTFA) as Polyquaternium 10 and available from Amerchol Corp. (Edison, N.J., USA).

### Cationically Modified Starch Polymer

The personal care compositions may also comprise a water-soluble cationically modified starch polymer. As used herein, the term "cationically modified starch" refers to a starch to which a cationic group is added prior to degradation of the starch to a smaller molecular weight, or wherein a cationic group is added after modification of the starch to achieve a desired molecular weight. The definition of the term "cationically modified starch" also includes amphoterically modified starch. The term "amphoterically modified starch" refers to a starch hydrolysate to which a cationic group and an anionic group are added.

The personal care compositions may comprise cationically modified starch polymers at a range of from about 0.01% to about 10%, and more preferably from about 0.05% to about 5%, by weight of the composition.

The cationically modified starch polymers disclosed herein have a percent of bound nitrogen of from about 0.5% to about 4%.

The cationically modified starch polymers also have a molecular weight of from about 50,000 to about 15,000,000. As used herein, the term "molecular weight" refers to the weight average molecular weight. The weight average molecular weight may be measured by gel permeation chromatography ("GPC") using a Waters 600E HPLC pump and Waters 717 auto-sampler equipped with a Polymer Laboratories PL Gel MIXED-A GPC column (Part Number 1110-6200, 600 x 7.5 mm, 20 µm) at a column temperature of 55ºC and at a flow rate of 1.0 ml/min (mobile phase consisting of Dimethylsulfoxide with 0.1% Lithium Bromide), and using a Wyatt DAWN EOS MALLS (multi-angle laser light scattering detector) and Wyatt Optilab DSP (interferometric refractometer) detectors arranged in series (using a dn/dc of 0.066), all at detector temperatures of 50ºC, with a method created by using a Polymer Laboratories narrow dispersed Polysaccharide standard (Mw = 47,300), with an injection volume of 200 µl.

The cationically modified starch polymers have a charge density at least about 3.0 meq/g. The chemical modification to obtain such a charge density includes, but is not limited to, the addition of amino and/or ammonium groups into the starch molecules. Non-limiting examples of these ammonium groups may include substituents such as hydroxypropyl trimmonium chloride, trimethylhydroxypropyl ammonium chloride, dimethylstearylhydroxypropyl ammonium chloride, and dimethyldodecylhydroxypropyl ammonium chloride. See Solarek, D. B., Cationic Starches in Modified Starches: Properties and Uses, Wurzburg, O.B., Ed., CRC Press, Inc., Boca Raton, Florida 1986, pp 113-125. The cationic groups may be added to the starch prior to degradation to a smaller molecular weight or the cationic groups may be added after such modification.

The cationically modified starch polymers generally have a degree of substitution of a cationic group that would result in a charge density of at least a 3.0 meq/g. As used herein, the "degree of substitution" of the cationically modified starch polymers is an average measure of the number of hydroxyl groups on each anhydroglucose unit which is derivatized by substituent groups. Since each anhydroglucose unit has three potential hydroxyl groups available for substitution, the maximum possible degree of substitution is 3. The degree of substitution is expressed as the number of moles of substituent groups per mole of anhydroglucose unit, on a molar average basis. The degree of substitution may be determined using proton nuclear magnetic resonance spectroscopy (¹H NMR) methods well known in the art. Suitable 1H NMR techniques include those described in "Observation on NMR Spectra of Starches in Dimethyl Sulfoxide, Iodine-Complexing, and Solvating in Water-Dimethyl Sulfoxide", Qin-Ji Peng and Arthur S. Perlin, Carbohydrate Research, 160 (1987), 57-72; and "An Approach to the Structural Analysis of Oligosaccharides by NMR Spectroscopy", J. Howard Bradbury and J. Grant Collins, Carbohydrate Research, 71, (1979), 15-25.

The cationically modified starch polymer may comprise maltodextrin. Thus, in one embodiment of the present invention, the cationically modified starch polymers may be further characterized by a Dextrose Equivalance ("DE") value of less than about 35, and more preferably from about 1 to about 20. The DE value is a measure of the reducing equivalence of the hydrolyzed starch referenced to dextrose and expressed as a percent (on dry basis). Starch completely hydrolyzed to dextrose has a DE value of 100, and unhydrolyzed starch has a DE value of 0. A suitable assay for DE value includes one described in "Dextrose Equivalent", Standard Analytical Methods of the Member Companies of the Corn Industries Research Foundation, 1st ed., Method E-26. Additionally, the cationically modified starch polymers may comprise a dextrin. Dextrin is typically a pyrolysis product of starch with a wide range of molecular weights.

The source of starch before chemical modification can be chosen from a variety of sources such as tubers, legumes, cereal, and grains. Non-limiting examples of this source starch may include corn starch, wheat starch, rice starch, waxy corn starch, oat starch, cassava starch, waxy barley, waxy rice starch, glutenous rice starch, sweet rice starch, amioca, potato starch, tapioca starch, oat starch, sago starch, sweet rice, or mixtures thereof. Waxy corn starch is preferred.

In one embodiment, cationically modified starch polymers are selected from degraded cationic maize starch, cationic tapioca, cationic potato starch, and mixtures thereof. In another embodiment, cationically modified starch polymers are cationic corn starch

The starch, prior to degradation or after modification to a smaller molecular weight, may comprise one or more additional modifications. For example, these modifications may include cross-linking, stabilization reactions, phophorylations, and hydrolyzations. Stabilization reactions may include alkylation and esterification.

The cationically modified starch polymers may be incorporated into the composition in the form of hydrolyzed starch (e.g., acid, enzyme, or alkaline degradation), oxidized starch (e.g., peroxide, peracid, hypochlorite, alkaline, or any other oxidizing agent), physically/mechanically degraded starch (e.g., via the thermomechanical energy input of the processing equipment), or combinations thereof.

Suitable cationically modified starch for use in compositions is available from known starch suppliers. Also suitable for use in the present invention is nonionic modified starch that could be futher derivatized to a cationically modified starch as is known in the art. Other suitable modified starch starting materials may be quaternized, as is known in the art, to produce the cationically modified starch polymer suitable for use in the invention.

### Detersive Surfactant

The personal care composition may include a detersive surfactant. The detersive surfactant is included to provide cleaning performance to the composition. The detersive surfactant may be selected from the group consisting of anionic detersive surfactants, zwitterionic or amphoteric surfactants, and combinations thereof. Such surfactants should be physically and chemically compatible with the essential components described herein, or should not otherwise unduly impair product stability, aesthetics or performance.

Suitable anionic detersive surfactants for use in the personal care composition include those which are known for use in hair care or other personal care cleansing compositions. The concentration of the anionic surfactant component in the composition should be sufficient to provide the desired cleaning and lather performance, and generally range from about 5% to about 50%, preferably from about 8% to about 30%, more preferably from about 10% to about 25%, even more preferably from about 12% to about 22%.

Preferred anionic detersive surfactants for use in the compositions include ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium lauryl sulfate, potassium laureth sulfate, sodium lauryl sarcosinate, sodium lauroyl sarcosinate, lauryl sarcosine, cocoyl sarcosine, ammonium cocoyl sulfate, ammonium lauroyl sulfate, sodium cocoyl sulfate, sodium lauroyl sulfate, potassium cocoyl sulfate, potassium lauryl sulfate, triethanolamine lauryl sulfate, triethanolamine lauryl sulfate, monoethanolamine cocoyl sulfate, monoethanolamine lauryl sulfate, sodium tridecyl benzene sulfonate, sodium dodecyl benzene sulfonate, sodium cocoyl isethionate and combinations thereof.

Suitable amphoteric or zwitterionic detersive surfactants for use in the composition herein include those which are known for use in hair care or other personal care cleansing. Concentrations of such amphoteric detersive surfactants preferably ranges from about 0.5% to about 20%, preferably from about 1% to about 10%. Non-limiting examples of suitable zwitterionic or amphoteric surfactants are described in U.S. Patent Nos. 5,104,646 (Bolich Jr. et al.), and 5,106,609 (Bolich Jr. et al.).

### Chemical Exfoliant

The personal care composition may include one or more chemical exfoliants. The exfoliant is included in order to remove dead skin and expose hair follicles in preparation for delivery of treatment actives herein. Non-limiting chemical exfoliants include salicylic acid, glycolic acid, enzymes, citric acid, malic acid, alpha hydroxyl acid (AHA's), beta hydroxyl acid, (BHA's) and mixtures thereof.

The chemical exfoliant may be present in the personal care composition at a level of from about 0.050% to about 10.0%, preferably from about 0.100% to about 8.00%, and most preferably from about 0.500% to about 5.00% by weight of the composition.

### Oily Conditioning Agent

In a preferred embodiment of the present invention, the personal care compositions comprise one or more oily conditioning agents. Oily conditioning agents include materials which are used to give a particular conditioning benefit to hair and/or skin. In hair treatment compositions, suitable conditioning agents are those which deliver one or more benefits relating to shine, softness, combability, antistatic properties, wet-handling, damage, manageability, body, and greasiness. The oily conditioning agents useful in the compositions of the present invention typically comprise a water-insoluble, water-dispersible, non-volatile, liquid that forms emulsified, liquid particles. Suitable oily conditioning agents for use in the composition are those conditioning agents characterized generally as silicones (e.g., silicone oils, cationic silicones, silicone gums, high refractive silicones, and silicone resins), organic conditioning oils (e.g., hydrocarbon oils, polyolefins, and fatty esters) or combinations thereof, or those conditioning agents which otherwise form liquid, dispersed particles in the aqueous surfactant matrix herein.

One or more oily conditioning agents are typically present at a concentration from about 0.01% to about 10%, preferably from about 0.1% to about 8%, more preferably from about 0.2% to about 4%, by weight of the composition.

### Silicone Conditioning Agent

The oily conditioning agents of the compositions are preferably a water-insoluble silicone conditioning agent. The silicone conditioning agent may comprise volatile silicone, non-volatile silicone, or combinations thereof. Preferred are non-volatile silicone conditioning agents. If volatile silicones are present, it will typically be incidental to their use as a solvent or carrier for commercially available forms of non-volatile silicone materials ingredients, such as silicone gums and resins. The silicone conditioning agent particles may comprise a silicone fluid conditioning agent and may also comprise other ingredients, such as a silicone resin to improve silicone fluid deposition efficiency or enhance glossiness of the hair.

Non-limiting examples of suitable silicone conditioning agents, and optional suspending agents for the silicone, are described in U.S. Reissue Pat. No. 34,584, U.S. Pat. No. 5,104,646, and U.S. Pat. No. 5,106,609. The silicone conditioning agents for use in the compositions preferably have a viscosity, as measured at 25°C, from about 20 to about 2,000,000 centistokes ("csk") [about 20 x 10⁻⁶ m²/s to about 2 m²/s], more preferably from about 1,000 to about 1,800,000 csk [about 0.001 m²/s to about 1.8 m²/s], even more preferably from about 5,000 to about 1,500,000 csk [about 0.005 m²/s to about 1.5 m²/s], more preferably from about 10,000 to about 1,000,000 csk [about 0.01 m²/s to about 1 m²/s].

In an exemplary opaque composition, the personal care composition comprises a non-volatile silicone oil having a particle size as measured in the personal care composition from about 1 µm to about 50 µm. In an embodiment of the present invention for small particle application to the hair, the personal care composition comprises a non-volatile silicone oil having a particle size as measured in the personal care composition from about 100 nm to about 1 µm. A substantially clear exemplary composition comprises a non-volatile silicone oil having a particle size as measured in the personal care composition of less than about 100 nm.

Non-volatile silicone oils suitable for use in compositions may be selected from organo-modified silicones and fluoro-modified silicones. In one embodiment of the present invention, the non-volatile silicone oil is an organo-modified silicone which comprises an organo group selected from the group consisting of alkyl groups, alkenyl groups, hydroxyl groups, amine groups, quaternary groups, carboxyl groups, fatty acid groups, ether groups, ester groups, mercapto groups, sulfate groups, sulfonate groups, phosphate groups, propylene oxide groups, and ethylene oxide groups.

In a preferred embodiment of the present invention, the non-volatile silicone oil is dimethicone.

Background material on silicones including sections discussing silicone fluids, gums, and resins, as well as manufacture of silicones, are found in Encyclopedia of Polymer Science and Engineering, vol. 15, 2d ed., pp 204-308, John Wiley & Sons, Inc. (1989).

Silicone fluids suitable for use in the compositions are disclosed in U.S. Pat. No. 2,826,551, U.S. Pat. No. 3,964,500, U.S. Pat. No. 4,364,837, British Pat. No. 849,433, and Silicon Compounds, Petrarch Systems, Inc. (1984).

### Organic Conditioning Oils

The oily conditioning agent of the compositions may also comprise at least one organic conditioning oil, either alone or in combination with other conditioning agents, such as the silicones described above.

### Hydrocarbon Oils

Suitable organic conditioning oils for use as conditioning agents in the compositions include, but are not limited to, hydrocarbon oils having at least about 10 carbon atoms, such as cyclic hydrocarbons, straight chain aliphatic hydrocarbons (saturated or unsaturated), and branched chain aliphatic hydrocarbons (saturated or unsaturated), including polymers and mixtures thereof. Straight chain hydrocarbon oils preferably are from about C₁₂ to about C₁₉. Branched chain hydrocarbon oils, including hydrocarbon polymers, typically will contain more than 19 carbon atoms.

Specific non-limiting examples of these hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, polybutene, polydecene, and mixtures thereof. Branched-chain isomers of these compounds, as well as of higher chain length hydrocarbons, can also be used, examples of which include 2, 2, 4, 4, 6, 6, 8, 8-dimethyl-10-methylundecane and 2, 2, 4, 4, 6, 6-dimethyl-8-methylnonane, available from Permethyl Corporation. A preferred hydrocarbon polymer is polybutene, such as the copolymer of isobutylene and butene, which is commercially available as L-14 polybutene from Amoco Chemical Corporation.

### Polyolefins

Organic conditioning oils for use in the compositions can also include liquid polyolefins, more preferably liquid poly-α-olefins, more preferably hydrogenated liquid poly-α-olefins. Polyolefins for use herein are prepared by polymerization of C₄ to about C₁₄ olefenic monomers, preferably from about C₆ to about C₁₂.

Non-limiting examples of olefenic monomers for use in preparing the polyolefin liquids herein include ethylene, propylene, 1-butene, 1-pentene, 1-hexene, 1-octene, 1-decene, 1-dodecene, 1-tetradecene, branched chain isomers such as 4-methyl-1-pentene, and mixtures thereof. Also suitable for preparing the polyolefin liquids are olefin-containing refinery feedstocks or effluents.

### Fatty Esters

Other suitable organic conditioning oils for use as the conditioning agent in the compositions include fatty esters having at least 10 carbon atoms. These fatty esters include esters with hydrocarbyl chains derived from fatty acids or alcohols. The hydrocarbyl radicals of the fatty esters hereof may include or have covalently bonded thereto other compatible functionalities, such as amides and alkoxy moieties (*e.g.,* ethoxy or ether linkages, *etc*.).

Specific examples of preferred fatty esters include, but are not limited to, isopropyl isostearate, hexyl laurate, isohexyl laurate, isohexyl palmitate, isopropyl palmitate, decyl oleate, isodecyl oleate, hexadecyl stearate, decyl stearate, isopropyl isostearate, dihexyldecyl adipate, lauryl lactate, myristyl lactate, cetyl lactate, oleyl stearate, oleyl oleate, oleyl myristate, lauryl acetate, cetyl propionate, and oleyl adipate.

Other fatty esters suitable for use in the compositions are those known as polyhydric alcohol esters. Such polyhydric alcohol esters include alkylene glycol esters.

Still other fatty esters suitable for use in the compositions are glycerides, including, but not limited to, mono-, di-, and tri-glycerides, preferably di- and tri-glycerides, more preferably triglycerides. A variety of these types of materials can be obtained from vegetable and animal fats and oils, such as castor oil, safflower oil, cottonseed oil, corn oil, olive oil, cod liver oil, almond oil, avocado oil, palm oil, sesame oil, lanolin and soybean oil. Synthetic oils include, but are not limited to, triolein and tristearin glyceryl dilaurate.

### Fluorinated Conditioning Compounds

Fluorinated compounds suitable for delivering conditioning to hair or skin as organic conditioning oils include perfluoropolyethers, perfluorinated olefins, fluorine based specialty polymers that may be in a fluid or elastomer form similar to the silicone fluids previously described, and perfluorinated dimethicones. Specific non-limiting examples of suitable fluorinated compounds include the Fomblin® product line from Ausimont which includes HC/04, HC/25, HC01, HC/02, HC/03; dioctyldodecyl fluoroeptyl citrate, commonly called Biosil Basics Fluoro Guerbet 3.5 supplied by Biosil Technologies; and Biosil Basics Fluorosil LF also supplied by Biosil Technologies.

### Fatty Alcohols

Other suitable organic conditioning oils for use in the personal care compositions include, but are not limited to, fatty alcohols having at least about 10 carbon atoms, more preferably about 10 to about 22 carbon atoms, most preferably about 12 to about 16 carbon atoms. Also suitable for use in the personal care compositions are alkoxylated fatty alcohols which conform to the general formula:

CH₃(CH₂)ₙCH₂(OCH₂CH₂)ₚOH

wherein n is a positive integer having a value from about 8 to about 20, preferably about 10 to about 14, and p is a positive integer having a value from about 1 to about 30, preferably from about 2 to about 23.

### Alkyl Glucosides and Alkyl Glucoside Derivatives

Suitable organic conditioning oils for use in the personal care compositions include, but are not limited to, alkyl glucosides and alkyl glucoside derivatives. Specific non-limiting examples of suitable alkyl glucosides and alkyl glucoside derivatives include Glucam E-10, Glucam E-20, Glucam P-10, and Glucquat 125 commercially available from Amerchol.

### Other Conditioning Agents

### Quaternary Ammonium Compounds

Suitable quaternary ammonium compounds for use as conditioning agents in the personal care compositions include, but are not limited to, hydrophilic quaternary ammonium compounds with a long chain substituent having a carbonyl moiety, like an amide moiety, or a phosphate ester moiety or a similar hydrophilic moiety.

Examples of useful hydrophilic quaternary ammonium compounds include, but are not limited to, compounds designated in the CTFA Cosmetic Dictionary as ricinoleamidopropyl trimonium chloride, ricinoleamido trimonium ethylsulfate, hydroxy stearamidopropyl trimoniummethylsulfate and hydroxy stearamidopropyl trimonium chloride, or combinations thereof.

Examples of other useful quaternary ammonium surfactants include, but are not limited to, Quaternium-33, Quaternium-43, isostearamidopropyl ethyldimonium ethosulfate, Quaternium-22 and Quaternium-26, or combinations thereof, as designated in the CTFA Dictionary.

Other hydrophilic quaternary ammonium compounds useful in a composition of the present invention include, but are not limited to, Quaternium-16, Quaternium-27, Quaternium-30, Quaternium-52, Quaternium-53, Quaternium-56, Quaternium-60, Quaternium-61, Quaternium-62, Quaternium-63, Quaternium-71, and combinations thereof.

### Polyethylene Glycols

Additional compounds useful herein as conditioning agents include polyethylene glycols and polypropylene glycols having a molecular weight of up to about 2,000,000 such as those with CTFA names PEG-200, PEG-400, PEG-600, PEG-1000, PEG-2M, PEG-7M, PEG-14M, PEG-45M and mixtures thereof.

### Additional Components

The personal care compositions may further comprise one or more additional components known for use in hair care or personal care products, provided that the additional components are physically and chemically compatible with the essential components described herein, or do not otherwise unduly impair product stability, aesthetics or performance. Individual concentrations of such additional components may range from about 0.001% to about 10% by weight of the personal care compositions.

Non-limiting examples of additional components for use in the composition include natural cationic deposition polymers, synthetic cationic deposition polymers, anti-dandruff agents, particles, suspending agents, paraffinic hydrocarbons, propellants, viscosity modifiers, dyes, non-volatile solvents or diluents (water-soluble and water-insoluble), pearlescent aids, foam boosters, additional surfactants or nonionic cosurfactants, pediculocides, pH adjusting agents, perfumes, preservatives, chelants, proteins, skin active agents, sunscreens, UV absorbers, and vitamins.

### Cellulose or Guar Cationic Deposition Polymers

The personal care compositions may also include cellulose or guar cationic deposition polymers. Cellulose or glactomannan cationic deposition polymers are preferred. Generally, such cellulose or guar cationic deposition polymers may be present at a concentration from about 0.05% to about 5%, by weight of the composition. Suitable cellulose or guar cationic deposition polymers have a molecular weight of greater than about 5,000. Preferably, the cellulose or guar cationic deposition polymers have a molecular weight of greater than about 200,000. Additionally, such cellulose or guar deposition polymers have a charge density from about 0.15 meq/g to about 4.0 meq/g at the pH of intended use of the personal care composition, which pH will generally range from about pH 3 to about pH 9, preferably between about pH 4 and about pH 8. The pH of the compositions are measured neat.

Suitable cellulose or guar cationic polymers include those which conform to the following formula: 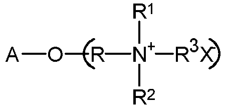 wherein A is an anhydroglucose residual group, such as a cellulose anhydroglucose residual; R is an alkylene oxyalkylene, polyoxyalkylene, or hydroxyalkylene group, or combination thereof; R¹, R², and R³ independently are alkyl, aryl, alkylaryl, arylalkyl, alkoxyalkyl, or alkoxyaryl groups, each group containing up to about 18 carbon atoms, and the total number of carbon atoms for each cationic moiety (i.e., the sum of carbon atoms in R¹, R² and R³) preferably being about 20 or less; and X is an anionic counterion. Non-limiting examples of such counterions include halides (e.g., chlorine, fluorine, bromine, iodine), sulfate and methylsulfate. The degree of cationic substitution in these polysaccharide polymers is typically from about 0.01 to about 1 cationic groups per anhydroglucose unit.

In one embodiment of the invention, the cellulose or guar cationic polymers are salts of hydroxyethyl cellulose reacted with trimethyl ammonium substituted epoxide, referred to in the industry (CTFA) as Polyquaternium 10 and available from Amerchol Corp. (Edison, N.J., USA).

### Synthetic Cationic Deposition Polymers

The personal care compositions may also include synthetic cationic deposition polymers. Generally, such synthetic cationic deposition polymers may be present at a concentration from about 0.025% to about 5%, by weight of the composition. Such synthetic cationic deposition polymers have a molecular weight from about 1,000 to about 5,000,000. Additionally, such synthetic cationic deposition polymers have a charge density from about 0.1 meq/g to about 5.0 mEq/g.

Suitable synthetic cationic deposition polymers include those which are water-soluble or dispersible, cationic, non-crosslinked, conditioning copolymers comprising: (i) one or more cationic monomer units; and (ii) one or more nonionic monomer units or monomer units bearing a terminal negative charge; wherein said copolymer has a net positive charge, a cationic charge density of from about 0.5 meq/g to about 10 meg/g, and an average molecular weight from about 1,000 to about 5,000,000.

Non-limiting examples of suitable synthetic cationic deposition polymers are described in United States Patent Application Publication US 2003/0223951 A1 to Geary et al.

### Anti-Dandruff Actives

The compositions may also contain an anti-dandruff active. Suitable non-limiting examples of anti-dandruff actives include pyridinethione salts, azoles, selenium sulfide, particulate sulfur, keratolytic agents, and mixtures thereof. Such anti-dandruff actives should be physically and chemically compatible with the essential components of the composition, and should not otherwise unduly impair product stability, aesthetics or performance.

Pyridinethione anti-microbial and anti-dandruff agents are described, for example, in U.S. Pat. No. 2,809,971; U.S. Pat. No. 3,236,733; U.S. Pat. No. 3,753,196; U.S. Pat. No. 3,761,418; U.S. Pat. No. 4,345,080; U.S. Pat. No. 4,323,683; U.S. Pat. No. 4,379,753; and U.S. Pat. No. 4,470,982.

Azole anti-microbials include imidazoles such as climbazole and ketoconazole.

Selenium sulfide compounds are described, for example, in U.S. Pat. No. 2,694,668; U.S. Pat. No. 3,152,046; U.S. Pat. No. 4,089,945; and U.S. Pat. No. 4,885,107.

Sulfur may also be used as a particulate anti-microbial/anti-dandruff agent in the anti-microbial compositions of the present invention.

The present invention may further comprise one or more keratolytic agents such as salicylic acid. In a preferred embodiment, salicylic acid provides chemical exfoliation activity.

Additional anti-microbial actives of the present invention may include extracts of *Melaleuca spp.* (tea tree) and charcoal.

When present in the composition, the anti-dandruff active is included in an amount from about 0.01% to about 5%, preferably from about 0.1% to about 3%, and more preferably from about 0.3% to about 2%, by weight of the composition.

### Particles

The compositions optionally may comprise particles. Preferably, particles useful in the present invention are dispersed water-insoluble particles. Particles useful in the present invention can be inorganic, synthetic, or semi-synthetic. In the compositions, it is preferable to incorporate no more than about 20%, more preferably no more than about 10% and even more preferably no more than 2%, by weight of the composition, of particles. In an embodiment of the present invention, the particles have an average mean particle size of less than about 300 *µ*m.

Non-limiting examples of inorganic particles include colloidal silicas, fumed silicas, precipitated silicas, silica gels, magnesium silicate, glass particles, talcs, micas, sericites, and various natural and synthetic clays including bentonites, hectorites, and montmorillonites.

Examples of synthetic particles include silicone resins, poly(meth)acrylates, polyethylene, polyester, polypropylene, polystyrene, polyurethane, polyamide (*e.g.*, Nylon®), epoxy resins, urea resins, acrylic powders, and the like.

Non-limiting examples of hybrid particles include sericite & cross-linked polystyrene hybrid powder, and mica and silica hybrid powder.

### Opacifying Agents

The compositions may also contain one or more opacifying agents. Opacifying agents are typically used in cleansing compositions to impart desired aesthetic benefits to the composition, such as color or pearlescence. In the compositions of the present invention, it is preferable to incorporate no more than about 20%, more preferably no more than about 10% and even more preferably no more than 2%, by weight of the composition, of opacifying agents.

Suitable opacifying agents include, for example, fumed silica, polymethylmethacrylate, micronized Teflon®, boron nitride, barium sulfate, acrylate polymers, aluminum silicate, aluminum starch octenylsuccinate, calcium silicate, cellulose, chalk, corn starch, diatomaceous earth, Fuller's earth, glyceryl starch, hydrated silica, magnesium carbonate, magnesium hydroxide, magnesium oxide, magnesium trisilicate, maltodextrin, microcrystaline cellulose, rice starch, silica, titanium dioxide, zinc laurate, zinc myristate, zinc neodecanoate, zinc rosinate, zinc stearate, polyethylene, alumina, attapulgite, calcium carbonate, calcium silicate, dextran, nylon, silica silylate, silk powder, soy flour, tin oxide, titanium hydroxide, trimagnesium phosphate, walnut shell powder, or mixtures thereof. The above mentioned powders may be surface treated with lecithin, amino acids, mineral oil, silicone oil, or various other agents either alone or in combination, which coat the powder surface and render the particles hydrophobic in nature.

The opacifying agents may also comprise various organic and inorganic pigments. The organic pigments are generally various aromatic types including azo, indigoid, triphenylmethane, anthraquinone, and xanthine dyes. Inorganic pigments include iron oxides, ultramarine and chromium or chromium hydroxide colors, and mixtures thereof.

### Suspending Agents

The compositions may further comprise a suspending agent at concentrations effective for suspending water-insoluble material in dispersed form in the compositions or for modifying the viscosity of the composition. Such concentrations generally range from about 0.1% to about 10%, preferably from about 0.3% to about 5.0%, by weight of the composition, of suspending agent.

Suspending agents useful herein include anionic polymers and nonionic polymers. Useful herein are vinyl polymers such as cross-linked acrylic acid polymers with the CTFA name Carbomer.

### Paraffinic Hydrocarbons

The compositions may contain one or more paraffinic hydrocarbons. Paraffinic hydrocarbons suitable for use in compositions include those materials which are known for use in hair care or other personal care compositions, such as those having a vapor pressure at 1 atm (101 kPa) of equal to or greater than about 21 °C (about 70°F). Non-limiting examples include pentane and isopentane.

### Propellants

The composition also may contain one or more propellants. Propellants suitable for use in compositions include those materials which are known for use in hair care or other personal care compositions, such as liquefied gas propellants and compressed gas propellants. Suitable propellants have a vapor pressure at 1 atm (101 kPa) of less than about 21 °C (about 70°F). Non-limiting examples of suitable propellants are alkanes, isoalkanes, haloalkanes, dimethyl ether, nitrogen, nitrous oxide, carbon dioxide, and mixtures thereof.

### Other Optional Components

The compositions may contain fragrance.

The compositions may also contain water-soluble and water-insoluble vitamins such as vitamins B1, B2, B6, B12, C, pantothenic acid, pantothenyl ethyl ether, panthenol, biotin and their derivatives, and vitamins A, D, E, and their derivatives. The compositions may also contain water-soluble and water-insoluble amino acids such as asparagine, alanine, indole, glutamic acid and their salts, and tyrosine, tryptamine, lysine, histadine and their salts.

The compositions may contain a mono- or divalent salt such as sodium chloride.

The compositions may also contain chelating agents.

The compositions may further comprise materials useful for hair loss prevention and hair growth stimulants or agents.

### Conditioner

The hair restorative blends herein may be provided as a leave-in conditioner. The conditioner composition comprises one or more conditioning actives. Preferably, the actives are natural or naturally derived actives selected from starches, guars, non-guar galactomannan polymer derivatives, plant extracts, and the like.

Starches suitable for the conditioner compositions are those which generally result from any vegetable source. Nonlimiting examples include corn, potato, the oats, rice, tapioca, the sorghum, the barley or corn.

The conditioning actives are used preferably in an amount of from 0.01 to 20% in weight compared to the total weight of the composition. More preferably, from 0.05 to 15% in weight compared to the total weight of the conditioner composition and even more preferably from 0.1 to 10% by weight of the composition.

The hair conditioning compositions may also comprise non-guar galactomannan polymer derivatives having a mannose to galactose ratio of greater than 2:1 on a monomer to monomer basis, the non-guar galactomannan polymer derivative is selected from the group consisting of a cationic non-guar galactomannan polymer derivative and an amphoteric non-guar galactomannan polymer derivative having a net positive charge. As used herein, the term "cationic non-guar galactomannan" refers to a non-guar galactomannan polymer to which a cationic group is added. The term "amphoteric non-guar galactomannan" refers to a non-guar galactomannan polymer to which a cationic group and an anionic group are added such that the polymer has a net positive charge. Non-guar galactomannan polymer derivatives provide improved efficacy of conditioning agents. Enhanced conditioning benefits include increased silicone deposition, which results in improved hair smoothness and combability. Further, the non-guar galactomannan polymer derivatives have been found to reduce overall viscosity of conditioning compositions, which results in improved feel benefits.

The gum for use in preparing the non-guar galactomannan polymer derivatives is typically obtained as naturally occurring material such as seeds or beans from plants. Examples of various non-guar galactomannan polymers include but are not limited to Tara gum (3 parts mannose / 1 part galactose), Locust bean or carob (4 parts mannose / 1 part galactose), and cassia gum (5 parts mannose / 1 part galactose). A preferred non-guar galactomannan polymer derivative is cationic cassia.

The cationic non-guar galactomannan polymer derivatives have a molecular weight from about 1,000 to about 10,000,000. In one embodiment of the present invention, the cationic non-guar galactomannan polymer derivatives have a molecular weight from about 5,000 to about 3,000,000. As used herein, the term "molecular weight" refers to the weight average molecular weight. The weight average molecular weight may be measured by gel permeation chromatography.

The hair conditioning compositions may include non-guar galactomannan polymer derivatives which have a cationic charge density from about 0.7 meq/g to about 7 meq/g. In one embodiment of the present invention, the non-guar galactomannan polymer derivatives have a charge density from about 0.9 meq/g to about 7 meq/g. The degree of substitution of the cationic groups onto the non-guar galactomannan structure should be sufficient to provide the requisite cationic charge density.

In one embodiment of the present invention, the non-guar galactomannan polymer derivative is a cationic derivative of the non-guar galactomannan polymer, which is obtained by reaction between the hydroxyl groups of the non-guar galactomannan polymer and reactive quaternary ammonium compounds

In another embodiment of the present invention, the non-guar galactomannan polymer derivative is an amphoteric non-guar galactomannan polymer derivative having a net positive charge, obtained when the cationic non-guar galactomannan polymer derivative further comprises an anionic group.

The hair conditioning compositions may comprise non-guar galactomannan polymer derivatives at a range of from about 0.01% to about 10%, and more preferably from about 0.05% to about 5%, by weight of the composition.

The conditioner compositions may further include one or more conditioning polymers selected from derivatives of cellulose ethers, quaternary derivatives of guar, homopolymers and copolymers of DADMAC, homopolymers and copolymers of MAPTAC and quaternary derivatives of starches. Specific examples, using the CTFA designation, include, but are not limited to Polyquaternium-10, Guar hydroxypropyltrimonium chloride, Starch hydroxypropyltrimonium chloride, Polyquaternium-4, Polyquaternium-5, Polyquaternium-6, Polyquaternium-7, Polyquaternium-14, Polyquaternium-15, Polyquaternium-22, Polyquaternium-24, Polyquaternium-28, Polyquaternium-32, Polyquaternium-33, Polyquaternium-36, Polyquaternium-37, Polyquaternium-39, Polyquaternium-45, Polyquaternium-47 and polymethacrylamidopropyltrimonium chloride, and mixtures thereof. When used, the conditioning polymers are preferably included in the conditioner composition at a concentration of from 0.1 to 10 weight percent, preferably from 0.2 to 6 weight percent and most preferably from 0.2 to 5 weight percent.

### Conditioning Agents

The conditioning compositions may also comprise one or more conditioning agents, such as those selected from the group consisting of cationic surfactants, cationic polymers, nonvolatile silicones (including soluble and insoluble silicones), nonvolatile hydrocarbons, saturated C14 to C22 straight chain fatty alcohols, nonvolatile hydrocarbon esters, and mixtures thereof. Preferred conditioning agents are cationic surfactants, cationic polymers, saturated C14 to C22 straight chain fatty alcohols, quarternary ammonium salts and silicones (especially insoluble silicones). Plant extracts such as ginseng root extract, silybaum marianum extract, phyllanthus emblica fruit extract, and the like are also suitable. The components hereof can comprise from about 0.1% to about 99%, more preferably from about 0.5% to about 90%, of conditioning agents. However, in the presence of an aqueous carrier, the conditioning agents preferably comprise from about 0.1% to about 90%, more preferably from about 0.5 to about 60% and most preferably from about 1% to about 50% by weight of the hair conditioning composition.

The conditioning compositions also include one or more natural stimulants in order to stimulate the scalp prior to application of the serum component. Exemplary natural stimulants include those such as ginseng and caffeine.

### Cationic Surfactants

Cationic surfactants, useful in the conditioner compositions, contain amino or quaternary ammonium moieties. The cationic surfactant will preferably, though not necessarily, be insoluble in the compositions hereof. Cationic surfactants among those useful herein are disclosed in the following documents: M.C. Publishing Co., McCutcheoris, Detergents Sc Emulsifiers, (North American edition 1979); Schwartz, et al., Surface Active Agents, Their Chemistry and Technology, New York: Interscience Publishers, 1949; U.S. Patent 3,155,591, Hilfer, issued November 3, 1964; U. S. Patent 3,929,678, Laughlin et al., issued December 30, 1975; U. S. Patent 3,959,461, Bailey et al., issued May 25, 1976; and U. S. Patent 4,387,090, Bolich, Jr., issued June 7, 1983. Among the quaternary ammonium-containing cationic surfactant materials useful herein are those of the general formula: 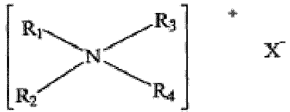 wherein R1-R4 are independently an aliphatic group of from about 1 to about 22 carbon atoms or an aromatic, alkoxy, polyoxyalkylene, alkylamido, hydroxyalkyl, aryl or alkylaryl group having from about 1 to about 22 carbon atoms; and X is a salt-forming anion such as those selected from halogen, (e.g. chloride, bromide), acetate, citrate, lactate, glycolate, phosphate nitrate, sulfate, and alkylsulfate radicals. The aliphatic groups may contain, in addition to carbon and hydrogen atoms, ether linkages, and other groups such as amino groups. The longer chain aliphatic groups, e.g., those of about 12 carbons, or higher, can be saturated or unsaturated. Especially preferred are di-long chain (e.g., di C12-22, preferably C14-C20, aliphatic, preferably alkyl) di-short chain (e.g., C1-C3 alkyl, preferably C1-C2 alkyl) and quaternary ammonium salts. Salts of primary, secondary and tertiary fatty amines are also suitable cationic surfactant materials. The alkyl groups of such amines preferably have from about 12 to about 22 carbon atoms, and may be substituted or unsubstituted. Such amines, useful herein, include stearamido propyl dimethyl amine, diethyl amino ethyl stearamide, dimethyl stearamine, dimethyl soyamine, soyamine, myristyl amine, tridecyl amine, ethyl stearylamine, N-tallowpropane diamine, ethoxylated (with 5 moles of ethylene oxide) stearylamine, dihydroxy ethyl stearylamine, and arachidylbehenylamine. Suitable amine salts include the halogen, acetate, phosphate, nitrate, citrate, lactate, and alkyl sulfate salts. Such salts include stearylamine hydrochloride, soyamine chloride, stearylamine formate, N-tallowpropane diamine dichloride and stearamidopropyl dimethylamine citrate. Cationic amine surfactants included among those useful in the present invention are disclosed in U.S. Patent 4,275,055, Nachtigal, et al., issued June 23, 1981. Cationic surfactants are preferably utilized at levels of from about 0.1% to about 10%, more preferably from about 0.25% to about 5%, most preferably from about 0.5% to about 2%, by weight of the composition.

### Serum

### In Vitro Experimental Data

In mammalian cells, testosterone is converted into the efficient steroid dihydrotestosterone (DHT) by 5α-reductase enzyme. An excess of DHT is one of the causes leading to sebaceous gland hypersecretion and/or to hair loss. The 5-α reductase type 1 is strongly expressed and active in keratinocytes, fibroblasts and sebaceous and apocrine glands, whereas the 5-α reductase type 2 is mainly localized in hair follicles and in the prostate.

The combination of Lichochalcone LR-15, Phlorogine, and Alpaflor Alp-Sebum was tested for their potential to inhibit 5-α reductase enzyme activity. A significant dose response was observed for this combination as compared to their respective performance individually.

### Method

Cells were seeded in a 24-well plate and cultured for 24 hours in culture medium. The medium was then replaced with assay medium containing the test compound, the association or the reference (finasteride at 1 x 10⁻⁵ M) and cells were pre-incubated for 24 hours. Cells were then treated with assay medium containing [¹⁴C]-testosterone and containing the test compound, the association or the reference and the cells were incubated for 24 hours. The culture supernatants were then collected for testosterone metabolism analysis and a standard MTT reduction assay was performed on the cell layers (cell viability assessment). All results are compared against a baseline control, which comprises water and no active compounds.
All experimental conditions were performed in n=3.

The steroid molecules from supernatants were extracted with a chloroform/methanol mix. The organic phase was collected and the different molecular species (testosterone metabolites) were separated by thin layer chromatography (TLC) and using a solvent system containing dichloromethane, ethyl acetate and methanol.

An autoradiography was performed on the chromatography and the transformed testosterone was estimated by densitometric analysis of the different spots corresponding to testosterone metabolites (Packard Cyclone PhosphorImager and Fujifilm Multigauge software).

At the end of incubation, the cells were incubated with MTT (tetrazolium salt) reduced in blue formazan crystals by succinate dehydrogenase (mitochondrial enzyme). This transformation is proportional to the enzyme activity. After cell dissociation and formazan crystal solubilization using DMSO, the optical density (OD) of the extracts at 540 nm, proportional to the number of living cells and their metabolic activity, was recorded with a microplate reader (VERSAmax, Molecular Devices). The equation used for measuring cell viability is as follows: viability (%) = (OD sample / OD control) x 100.

### Results

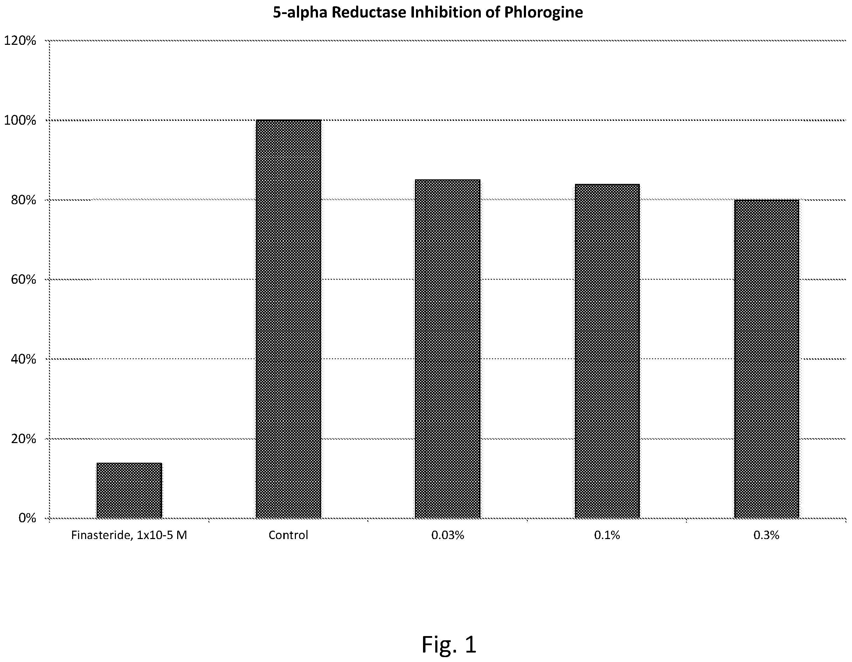

Fig. 1 shows the 5α-reductase inhibition profile for Phlorogine CV at 0.03%, 0.1% and 0.3% concentration levels, respectively. At its greatest concentration of 0.3%, the inhibitory effect is observed at 20% of the control (80% DHT expression detected). A tabular representation of the graph of Fig. 1 is provided below:

| **Table 1** | |
|---|---|
| **Phlorogine** | **DHT/Testosterone ratio as % of Control** |
| 0.03%* | 85% |
| 0.1%* | 84% |
| 0.3%* | 80% |

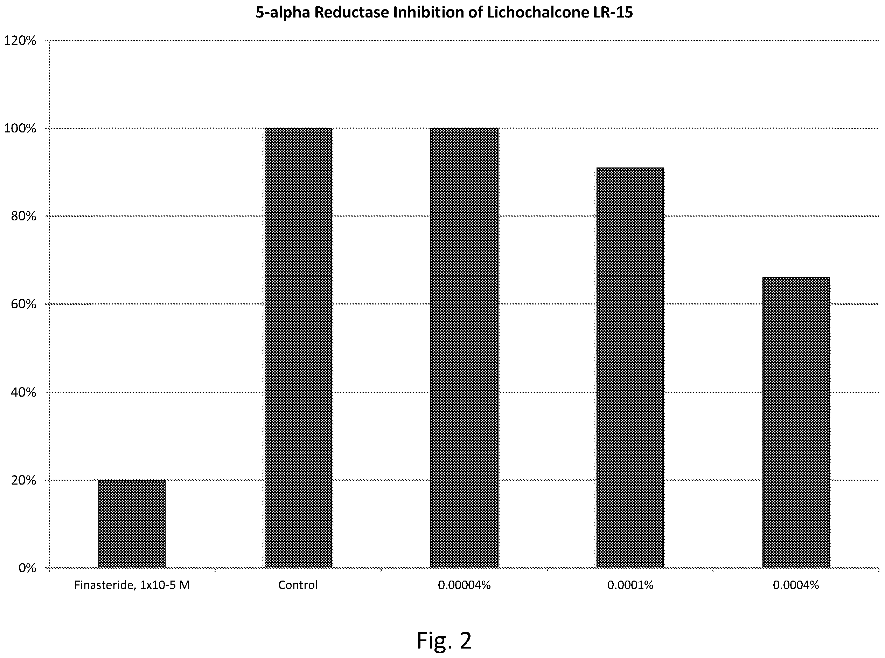

Fig. 2 shows the 5α-reductase inhibition profile for Licochalcone LR-15 at 0.00004%, 0.0001%, and 0.0004% concentration levels, respectively. At its greatest concentration of 0.0004%, the inhibitory effect is observed at 34% of the control (66% DHT expression detected). A tabular representation of the graph of Fig. 1 is provided below:

| **Table 2** | |
|---|---|
| **Lichochalcone LR-15** | **DHT/Testosterone ratio as % of Control** |
| 0.00004% | NA (Parity with Control) |
| 0.0001% | 91% |
| 0.0004% | 66% |

It was found that concentration levels in excess of 0.0004% became impeded by dramatic declines in cell viability. For example, at a concentration of 0.0004, mean cell viability is maintained at 108% of the negative control. But at a concentration of 0.0011%, cell viability is reduced to a mean of only 55% of the negative control. And as concentrations increased, mean cell viability is reduced to less than about 30% due to increasing cytotoxicity. And as cell viability degrades, DHT expression becomes moot due to a lack of cellular viability.

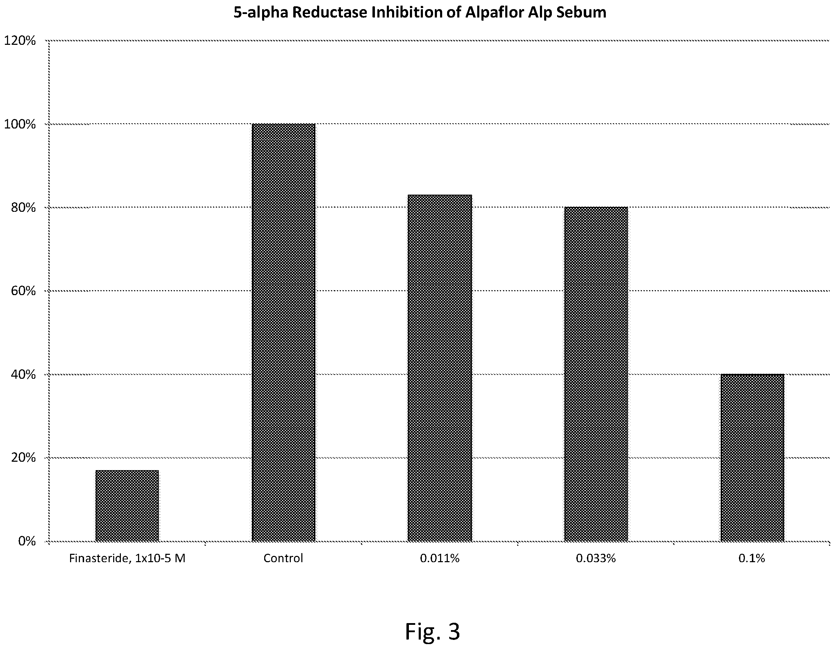

Fig. 3 shows the 5α-reductase inhibition profile for Alpaflor Alp-Sebum at 0.011%, 0.033%, and 0.100% concentration levels, respectively. At its greatest concentration of 0.1%, the inhibitory effect is observed at 40% of the control (60% DHT expression detected). A tabular representation of the graph of Fig. 3 is provided below:

| **Table 3** | |
|---|---|
| **Alpaflor Alp-Sebum** | **DHT/Testosterone ratio as % of Control** |
| 0.011% | 83% |
| 0.033% | 80% |
| 0.100% | 40% |

It was found that concentration levels in excess of 0.100% became impeded by dramatic declines in cell viability. For example, at a concentration of 0.1, mean cell viability is maintained at 92% of Control. But at a concentration of 0.37, cell viability is reduced to a mean of only 55% of Control. And as concentrations increased, mean cell viability is generally reduced to less than about 40% due to increasing cytotoxicity. And as cell viability degrades, DHT expression becomes moot due to a lack of cellular viability.

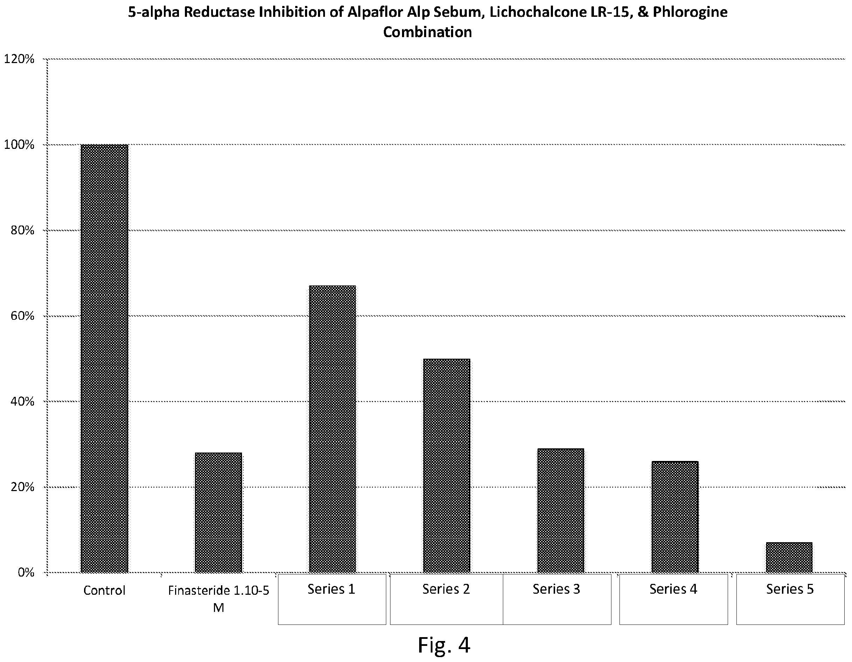

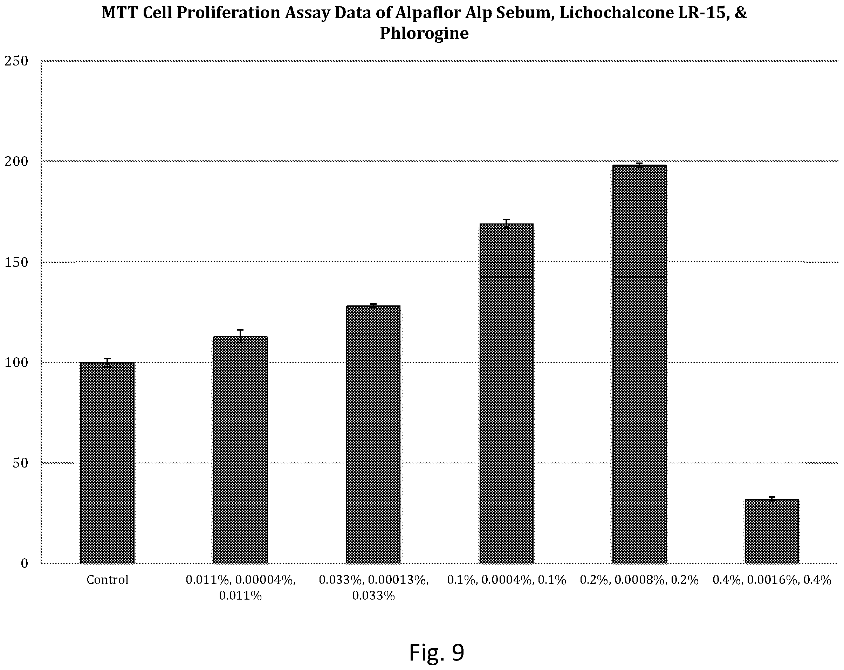

Fig. 4 shows the 5α-reductase inhibition profile of representative samples of the hair retention blend herein. A key, defining each "Series", giving the percent concentrations of each active within each of the "Series" of Fig. 4 and Fig. 9 is provided below:

| Ingredient | Series 1 | Series 2 | Series 3 | Series 4 | Series 5 |
|---|---|---|---|---|---|
| **Alpaflor Alp-Sebum** | 0.011% | 0.033% | 0.1% | 0.2% | 0.4% |
| **Phlorogine** | 0.011% | 0.033% | 0.1% | 0.2% | 0.4% |
| **Lichochalcone LR-15** | 0.00004% | 0.00013% | 0.0004% | 0.0008% | 0.0016% |

The 5α-reductase inhibition results provided in Fig. 4 are provided below in a tabular format below:

| **Table 4** | |
|---|---|
| **DHT/Testosterone, % Control** | **DHT/Testosterone ratio as % of Control** |
| Control | 100% |
| Finasteride 1x10⁻⁵ M | 28% |
| Series 1 | 67% |
| Series 2 | 50% |
| Series 3 | 29% |
| Series 4 | 26% |
| Series 5 | 7% |

As is shown in Fig. 4, and Table 4 above, Series 5 demonstrated a 93% inhibition of 5α-reductase (7% 5α-reductase expression). As such efficacy could not be achieved by each of the results shown in Figs. 1-3, it is clear that the combined hair retention blend exhibits a synergistic effect. And as will be demonstrated hereinafter, the cytotoxicity and loss of cell viability suffered with increasing concentrations of the individual assays of Figs. 1-3 is not present in the hair restorative blend of Fig. 4. In fact, as is discussed hereinafter, increased concentrations of the hair restorative blends of Fig. 4 demonstrate increased cell proliferation and cell viability. Dermal papilla cell viability is measured with a standard MTT assay (via ASTM E2526 - 08(2013)).

Figs. 5-8 illustrate dermal papilla cell viability in response to increasing active concentrations of individual 5α-reductase inhibitors and the hair restorative blend of 5α-reductase inhibitors.

Fig. 5 shows cell proliferation of Lichochalcone LR-15 at increasing concentration levels. A tabular representation of the results of Fig. 5 is provided at Table 5, below:

| Table 5 | |
|---|---|
| **Lichochalcone LR-15** | MTT (Viability) |
| Control | 100 |
| 0.000014% | 111 |
| 0.000041% | 115 |
| 0.0001% | 114 |
| 0.0004% | 108 |
| 0.0011% | 55 |
| 0.003% | 2 |
| 0.01% | 9 |
| 0.03% | 31 |

Fig. 6 shows cell proliferation of Alpaflor Alp-Sebum at increasing concentration levels. A tabular representation of the results of Fig. 6 is provided at Table 6. below:

| **Table 6** | |
|---|---|
| **Alpaflor Alp-Sebum** | MTT (Viability) |
| Control | 100 |
| 0.005% | 97 |
| 0.014% | 96 |
| 0.040% | 102 |
| 0.123% | 92 |
| 0.37% | 55 |
| 1.1% | 29 |
| 3.3% | 42 |
| 10.0% | 69 |

Fig. 7 shows cell proliferation of Phlorogine at increasing concentration levels. A tabular representation of the results of Fig. 7 is provided at Table 7 below:

| Table 7 | |
|---|---|
| Phlorogine | MTT (Viability) |
| 0.03% | 99 |
| 0.1% | 100 |
| 0.3% | 98 |

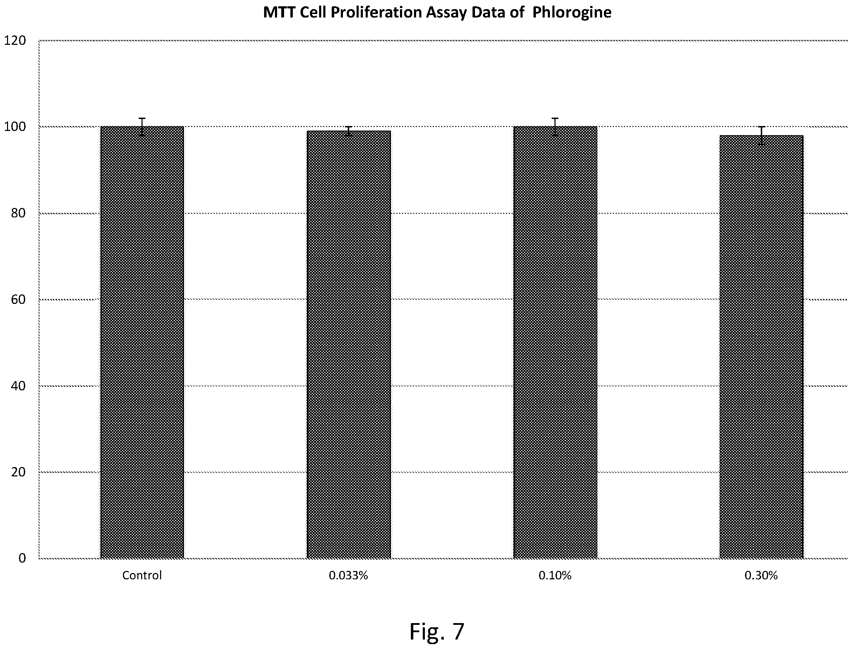

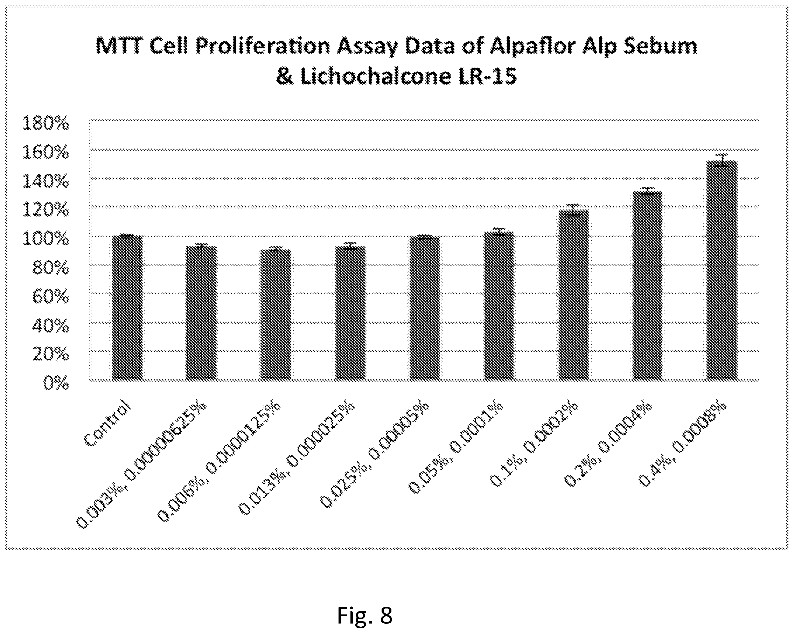

As is illustrated by Fig.7 and Table 7, Phlorogine does not have an apparent effect on cell proliferation in relation to its concentration. Therefore, the combination of Alpaflor Alp-Sebum and Lichochalcone LR-15 was tested to determine the effect of their interaction on cell viability. The results are provided in Fig. 8 and Table 8, discussed below.

Fig. 8 shows cell proliferation of the combination of Alpaflor Alp-Sebum and Lichochalcone LR-15 at increasing concentration levels. A tabular representation of the results of Fig. 8 is provided at Table 8 below:

| Table 8 | | |
|---|---|---|
| Alpaflor Alp-Sebum | Lichochalcone LR-15 | MTT (Viability) |
| Control | | 100% |
| 0.003% | 0.00000625% | 93% |
| 0.006% | 0.0000125% | 91% |
| 0.013% | 0.000025% | 93% |
| 0.025% | 0.00005% | 99% |
| 0.05% | 0.0001% | 103% |
| 0.1% | 0.0002% | 118% |
| 0.2% | 0.0004% | 131% |
| 0.4% | 0.0008% | 152% |

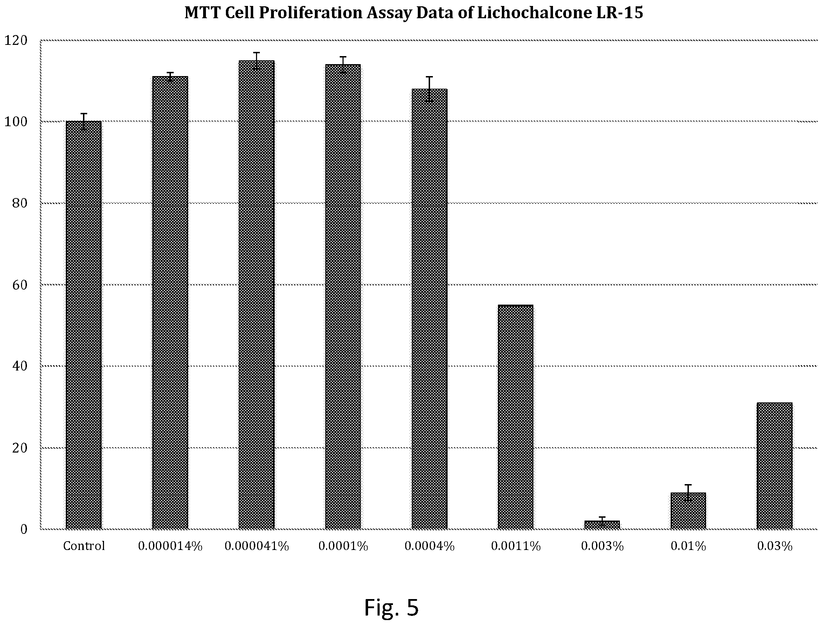

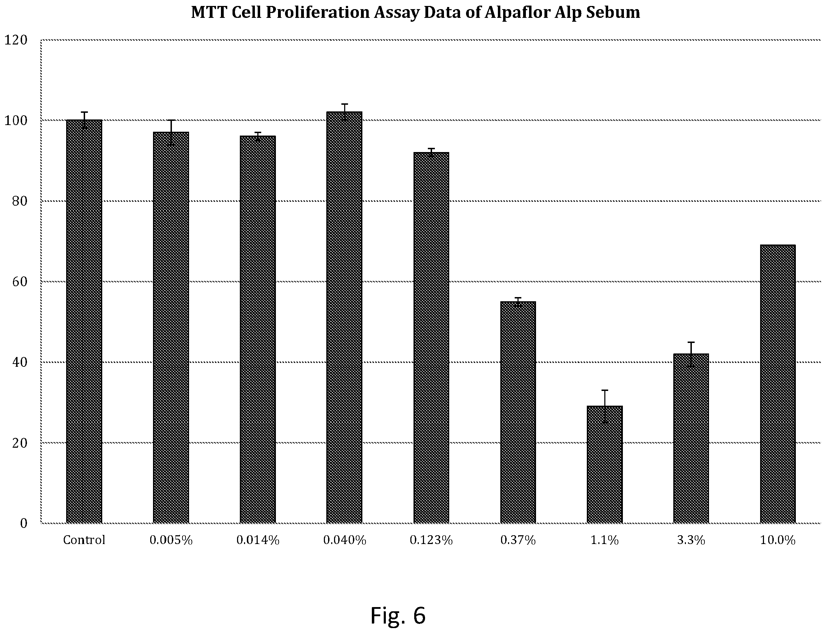

As is illustrated in Fig. 8 and Table 8 above, the combination of Alpaflor Alp-Sebum and Lichochalcone LR-15 not only avoid the cytotoxicity observed by the individual assays of Figs. 5 and 6, but cell proliferation is observed at levels of 118%, 131% and 152% as concentrations increased. And while Phlorogine is observed to be generally inert as to cell viability, it does not appear to be capable of cell proliferation at Fig. 7. Therefore, a clear synergy is observed by the combination of Alpaflor Alp-Sebum and Lichochalcone LR-15, observed in Fig. 8.

The cell proliferation results provided in Fig. 9 are provided below in a tabular format below:

| **Table 9** | |
|---|---|
| **DHT/Testosterone, % Control** | MTT (Viability) |
| **Control** | 100 |
| **Finasteride 1x10-5 M** | 101 |
| **Series 1** | 113 |
| **Series 2** | 128 |
| **Series 3** | 169 |
| **Series 4** | 198 |
| **Series 5** | 32 |

As is illustrated in Fig. 9 and Table 9 above, the hair restorative blends not only avoid the cytotoxicity observed by the individual assays of Figs. 5 and 6, but cell proliferation is observed at Series 2-4. And while Phlorogine is observed to be generally inert as to cell viability, it does not appear to be capable of cell proliferation at Fig. 7. Therefore, a clear synergy is observed by the hair restorative blend observed at Fig. 9.

### EXAMPLES

The following nonlimiting examples illustrate personal care compositions comprising the hair restorative blends herein. The compositions illustrated in the following Examples illustrate specific embodiments of the compositions used in the present invention, but are not intended to be limiting thereof. Other modifications can be undertaken by the skilled artisan without departing from the spirit and scope of this invention. These exemplified embodiments of the composition used in the present invention provide enhanced deposition of conditioning agents to the hair and/or skin.

All parts, percentages, and ratios herein are by weight unless otherwise specified. Some components may come from suppliers as dilute solutions. The levels given reflect the weight percent of the active material, unless otherwise specified.

The compositions illustrated in the following Examples are prepared by conventional formulation and mixing methods, an example of which is described above. All exemplified amounts are listed as weight percents and exclude minor materials such as diluents, preservatives, color solutions, imagery ingredients, botanicals, and so forth, unless otherwise specified.

| **Hair Restorative Blend** | **I.** | **II.** | **III.** | **IV.** |
|---|---|---|---|---|
| **Alpaflor Alp-Sebum¹** | 45 | 40 | 48 | 35 |
| **Licochalcone LR-15²** | 10 | 20 | 4 | 30 |
| **Phlorogine³** | 45 | 40 | 48 | 35 |

| | | | | |
|---|---|---|---|---|
| ¹Available from Centerchem ²Available from Barnet Products Corp *³Laminaria saccharina* Extract Available from BiotechMarine | | | | |

The hair restorative blends, exemplified above (I.-IV.) may then be incorporated into the various personal care compositions exemplified below. The hair retention blends will be designated as "HRB I.-IV.", respectively, in the examples hereinafter.

The following are representative of leave-in serum compositions of the present invention:

| **Serum Examples** | **1** | **2** | **3** | **4** | **5** | **6** | **7** | **8** |
|---|---|---|---|---|---|---|---|---|
| **WATER\AQUA\EAU** | q.s. | q.s. | q.s. | q.s. | q.s. | q.s. | q.s. | q.s. |
| **Ethanol** | 65 | 70 | 55 | 60 | 65 | 65 | 65 | 65 |
| **Acetyl-L-carnitine** | 0.009 | 0.01 | 0.015 | 0.008 | 0.007 | 0.02 | 0.017 | 0.01 |
| **5-AMP, free acid J⁴** | 0.04 | 0.04 | 0.04 | 0.04 | 0.04 | 0.04 | 0.04 | 0.04 |
| **Turmeric powder** | 0.05 | 0.05 | 0.05 | 0.05 | 0.05 | 0.05 | 0.05 | 0.05 |
| ***Emblica officinalis* fruit** | 0.05 | 0.03 | - | - | 0.05 | 0.04 | 0.05 | 0.05 |
| ***Panicum miliaceum* seed extract⁵** | 0.001 | 0.001 | - | - | - | - | - | - |
| ***Panax ginseng* 80%⁶** | 0.1 | - | 0.1 | - | 0.1 | 0.1 | - | - |
| **Green Tea Extract** | 0.05 | 0.05 | 0.05 | 0.05 | 0.05 | 0.05 | 0.05 | 0.05 |
| **Vitamin E** | 0.2 | 0.2 | 0.2 | 0.2 | 0.2 | 0.2 | 0.2 | 0.2 |
| **D/L-α Tocopheryl nicotinate** | 0.2 | 0.2 | 0.2 | 0.2 | 0.2 | 0.2 | 0.2 | 0.2 |
| **L-Arginine** | 0.1 | 0.1 | 0.1 | 0.1 | 0.1 | 0.1 | 0.1 | 0.1 |
| **Chlorellagen Del/ dermoch (*Chlorella vulgaris*)** | 0.5 | 0.5 | 0.5 | 0.5 | 0.5 | 0.5 | 0.5 | 0.5 |
| **Regenasure (glucosamine)** | 0.55 | 0.55 | 0.55 | 0.55 | 0.55 | 0.55 | 0.55 | 0.55 |
| **HRB I.** | 2.2 | 2.5 | | | | | | |
| **HRB II.** | | | 2.0 | 3.0 | | | | |
| **HRB III.** | | | | | 3.00 | 1.5 | | |
| **HRB IV.** | | | | | | | 3.3 | 4.0 |

| | | | | | | | | |
|---|---|---|---|---|---|---|---|---|
| ⁴Adenosine-5'-monophosphate free acid available from Sigma-Aldrich ⁵Available from FLAVEX Naturextrakte ⁶Available from Sunrich Chemical Zhuhai Co. Ltd. | | | | | | | | |

Serum Example 1, above was further tested to confirm *in vivo* efficacy of the hair restorative blend herein. The study protocol is provided below:

25 participants were instructed to wash hair, with a provided uniform shampoo, once daily. After washing hair, participants were instructed to apply the serum of Example 1 to the scalp, at uniform dosages. At 4, 8, and 12 weeks of use, hair is uniformly combed by participants, and hair lost due to comb-out is collected and recorded. Participants are trained on a uniform combing and collection technique. All results were tracked over a 12 week period.

After 12 weeks, hair retention data from the 25 participants was aggregated and a mean result is provided at the table below:

| **Table 10** | | | | | | | | |
|---|---|---|---|---|---|---|---|---|
| **TIMEPOINT (Weeks)** | **Breakage** | **% CHNG** | | **Breakage /PARTIAL BULB** | **% CHNG** | | **FULL BULB** | **% CHNG** |
| 0 | 22.00 | -- | | 53.65 | -- | | 26.43 | -- |
| 4 | 22.39 | 1.78% | | 77.39 | 44.25% | | 7.26 | -72.53% |
| 8 | 22.91 | 4.15% | | 57.57 | 7.29% | | 5.22 | -80.26% |
| 12 | 12.70 | -42.29% | | 81.30 | 51.54% | | 6.17 | -76.64% |

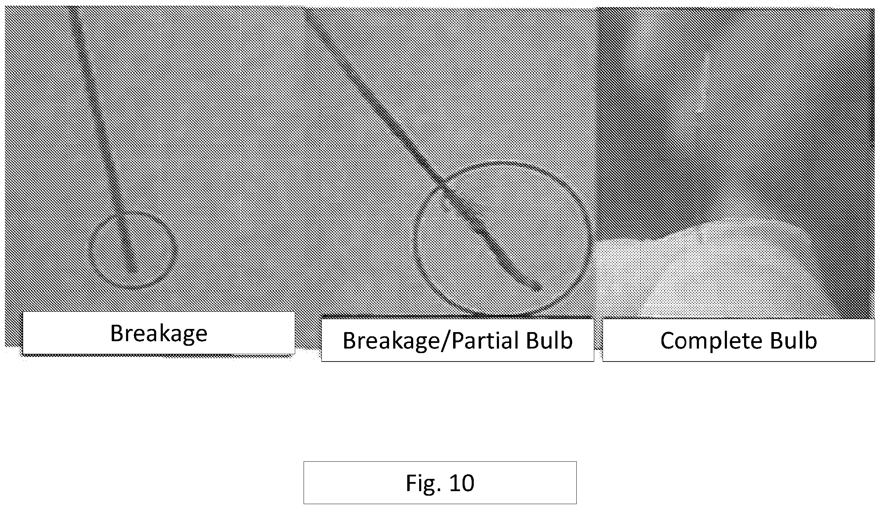

Table 10 shows the breakage counts illustrated by Fig. 10. Fig. 10 shows representations of 3 different breakage types which were measured during the uniform combing measurements at weeks 0, 4, 8, and 12 of the assessment of the serum of Example 1. The first category measured is designated as "Breakage". Breakage occurs when a strand of hair shows evidence of breaking anywhere along the hair strand above its root or bulb. This is typical of hair wear and tear.. The second category measured is designated "Breakage/Partial Bulb". The breakages of the second category show fracturing along the hair shaft but also include at least a portion of the hair root or bulb. Partial or atrophied bulbs are characteristic of hair follicles in exogen, which can be easily pulled out through combing or pushed out of the hair follicle when a new anagen hair growth phase is stimulated. The third category is designated as "Complete Bulb", which means that the hair did not break along its shaft, but instead includes a whole bulb or root, indicative of follicles in anagen or telogen phases of the hair cycle. The decrease in loss of full bulbs demonstrates a decrease in hair follicles entering into the telogen phase and maintenance or promotion of the growth (anagen) phase.

As is shown at Table 10, breakage was steadily reduced across all measured categories through week 12 of the assessment. Particularly, the "Complete Bulb" category showed a 76.64% improvement over week 0 measurements. This strongly correlates to the *in vitro* assays, which demonstrated strong 5-alpha reductase activity and dermal papilla cell propagation for the *in vitro* concentrations (Series 1-5) of the hair restorative blends herein. Accordingly, the commercial formulations exemplified here are correlated to the in vitro concentration distributions provided at Series 1-5 herein.

The following are representative of conditioner compositions that may be used in the present invention:

| **Conditioner Examples** | **9** | **10** | **11** | **12** | **13** | **14** | **15** | **16** |
|---|---|---|---|---|---|---|---|---|
| **WATER\AQUA\EAU** | q.s. | q.s. | q.s. | q.s. | q.s. | q.s. | q.s. | q.s. |
| **STEARALKONIUM CHLORIDE** | 1.50 | 1.50 | 1.50 | 1.50 | 1.50 | 1.50 | 1.50 | 1.50 |
| **CETYL ALCOHOL** | 4.00 | 4.00 | 4.00 | 4.00 | 4.00 | 4.00 | 4.00 | 4.00 |
| **GLYCERYL STEARATE** | 1.80 | 1.80 | 1.80 | 1.80 | 1.80 | 1.80 | 1.80 | 1.80 |
| **GLYCERYL STEARATE/ PEG-100 STEARATE** | 1.00 | 1.00 | 1.00 | 1.00 | 1.00 | 1.00 | 1.00 | 1.00 |
| **DISTEARYLDIMONIUM CHLORIDE** | 0.70 | 0.70 | 0.70 | 0.70 | 0.70 | 0.70 | 0.70 | 0.70 |
| **POLYQUATERNIUM-4** | 0.50 | 0.50 | 0.50 | 0.50 | 0.50 | 0.50 | 0.50 | 0.50 |
| **CETRIMONIUM CHLORIDE** | 2.00 | 2.00 | 2.00 | 2.00 | 2.00 | 2.00 | 2.00 | 2.00 |
| **DIMETHICONE PEG-8 POLYACRYLATE** | 0.25 - 3.00 | 0.25 - 3.00 | 0.25 - 3.00 | 0.25 - 3.00 | 0.25 - 3.00 | 0.25 - 3.00 | 0.25 - 3.00 | 0.25 - 3.00 |
| **HRB I.** | 5.00 | 5.00 | | | | | | |
| **HRB II.** | | | 8.00 | 10.00 | | | | |
| **HRB III.** | | | | | 3.00 | 14.00 | | |
| **HRB IV.** | | | | | | | 4.00 | 12.00 |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A non-therapeutic method for treating the appearance of thinning hair of a human comprising the steps of:
a. cleaning said hair with a shampoo composition;
b. rinsing said shampoo from said hair; and
c. applying a hair restorative blend to the scalp of said human, said hair restorative blend comprising:
i. a chalconoid;
ii. an *Epilobium* extract selected from *Epilobium fleischeri* extract or *Epilobium angustifolium* extract; and
iii. a cosmetically acceptable carrier.

2. The non-therapeutic method of claim 1, wherein the hair restorative blend further comprises a *Laminaria* extract, wherein said *Laminaria* extract is selected from the group consisting of *Laminaria saccharina* extract, *Laminaria digitata* extract, and *Laminaria ochroleuca* extract.

3. A non-therapeutic method according to claim 1, wherein the hair restorative blend further comprises a *Laminaria saccharina* extract present at a level of from 30% to 60% by weight of said hair restorative blend.

4. A non-therapeutic method according to claim 1, wherein said chalconoid is present at a level of from 0.1% to 30% by weight of said hair restorative blend.

5. A non-therapeutic method according to claim 1, wherein said chalconoid is selected from the group consisting of chalcone, butein, cardamomin, isoliquiritigenin, licochalcone A, licochalcone B, licochalcone C, licochalcone D, licochalcone E, sophoradin, xanthohumol, methyl hydroxychalcone, okanin, xanthohumol, and mixtures thereof.

6. A non-therapeutic method according to claim 1, wherein said *Epilobium* extract is present at a level of from 30% to 50% by weight of said hair restorative blend.

7. A non-therapeutic method according to claim 1, wherein said carrier is present at a level of from 60% to 85% by weight of the hair restorative blend.

8. A non-therapeutic method according to claim 1, wherein said hair restorative blend further comprises a cationically modified starch polymer.

9. A non-therapeutic method according to claim 1, wherein said shampoo comprises a detersive surfactant and a chemical exfoliant.

10. A non-therapeutic method according to claim 1, wherein said shampoo composition comprises an oily conditioning agent.

## Patentansprüche

1. Nicht therapeutisches Verfahren zum Behandeln des Erscheinens von ausdünnendem Haar eines Menschen, das die Schritte umfasst des:
a. Reinigens des Haars mit einer Shampoozusammensetzung;
b. Spülens des Shampoos aus dem Haar; und
c. Auftragens eines Haar stärkenden Gemischs auf die Kopfhaut des Menschen, wobei das Haar stärkende Gemisch umfasst:
i. ein Chalconoid;
ii. einen *Epilobium*-Extrakt, der aus *Epilobium-fleischeri-*Extrakt oder *Epilobium-angustifolium-*Extrakt ausgewählt ist; und
iii. einen kosmetisch akzeptablen Träger.

2. Nicht therapeutisches Verfahren nach Anspruch 1, wobei das Haar stärkende Gemisch weiter einen *Laminaria*-Extrakt umfasst, wobei der *Laminaria*-Extrakt aus der Gruppe ausgewählt ist, die aus *Laminaria-saccharina*-Extrakt*, Laminaria-digitata*-Extrakt und *Laminaria-ochroleuca*-Extrakt besteht.

3. Nicht therapeutisches Verfahren nach Anspruch 1, wobei das Haar stärkende Gemisch weiter *Laminaria-saccharina*-Extrakt umfasst, der mit einem Niveau von 30 Gew.-% bis 60 Gew.-% des Haar stärkenden Gemischs vorliegt.

4. Nicht therapeutisches Verfahren nach Anspruch 1, wobei das Chalconoid mit einem Niveau von 0,1 Gew.-% bis 30 Gew.-% des Haar stärkenden Gemischs vorliegt.

5. Nicht therapeutisches Verfahren nach Anspruch 1, wobei das Chalconoid aus der Gruppe ausgewählt ist, die aus Chalcon, Butein, Cardamomin, Isoliquiritigenin, Licochalcon A, Licochalcon B, Licochalcon C, Licochalcon D, Licochalcon E, Sophoradin, Xanthohumol, Methylhydroxychalcon, Okanin, Xanthohumol und Gemischen davon besteht.

6. Nicht therapeutisches Verfahren nach Anspruch 1, wobei der *Epilobium*-Extrakt mit einem Niveau von 30 Gew.-% bis 50 Gew.-% des Haar stärkenden Gemischs vorliegt.

7. Nicht therapeutisches Verfahren nach Anspruch 1, wobei der Träger mit einem Niveau von 60 Gew.-% bis 85 Gew.-% des Haar stärkenden Gemischs vorliegt.

8. Nicht therapeutisches Verfahren nach Anspruch 1, wobei das Haar stärkende Gemisch weiter ein kationisch modifiziertes Stärkepolymer umfasst.

9. Nicht therapeutisches Verfahren nach Anspruch 1, wobei das Shampoo ein reinigendes Tensid und ein chemisches Peeling umfasst.

10. Nicht therapeutisches Verfahren nach Anspruch 1, wobei die Shampoozusammensetzung eine ölhaltige Spülung umfasst.

## Revendications

1. Procédé non thérapeutique pour traiter l'apparence d'une chevelure clairsemée chez un humain, comprenant les étapes de :
a. nettoyage de ladite chevelure avec une composition de shampooing ;
b. rinçage dudit shampooing de ladite chevelure ; et
c. application d'un mélange de régénération capillaire sur le cuir chevelu dudit humain, ledit mélange de régénération capillaire comprenant :
i. un chalconoïde ;
ii. un extrait d'*Epilobium* choisi parmi un extrait d'*Epilobium fleischeri* ou un extrait d'*Epilobium angustifolium ;* et
iii. un vecteur cosmétiquement acceptable.

2. Procédé non thérapeutique selon la revendication 1, dans lequel le mélange de régénération capillaire comprend en outre un extrait de *Laminaria,* dans lequel ledit extrait de *Laminaria* est choisi parmi le groupe consistant en un extrait de *Laminaria saccharina,* un extrait de *Laminaria digitata* et un extrait de *Laminaria ochroleuca.*

3. Procédé non thérapeutique selon la revendication 1, dans lequel le mélange de régénération capillaire comprend en outre un extrait de *Laminaria saccharina* présent à un taux allant de 30 % à 60 % en poids dudit mélange de régénération capillaire.

4. Procédé non thérapeutique selon la revendication 1, dans lequel ledit chalconoïde est présent à un taux allant de 0,1 % à 30 % en poids dudit mélange de régénération capillaire.

5. Procédé non thérapeutique selon la revendication 1, dans lequel ledit chalconoïde est choisi parmi le groupe consistant en chalcone, butéine, cardamomine, isoliquiritigénine, licochalcone A, licochalcone B, licochalcone C, licochalcone D, licochalcone E, sophoradine, xanthohumol, méthylhydroxychalcone, okanine, xanthohumol et des mélanges de ceux-ci.

6. Procédé non thérapeutique selon la revendication 1, dans lequel ledit extrait d'*Epilobium* est présent à un taux allant de 30% à 50% en poids dudit mélange de régénération capillaire.

7. Procédé non thérapeutique selon la revendication 1, dans lequel ledit vecteur est présent à un taux allant de 60 % à 85 % en poids du mélange de régénération capillaire.

8. Procédé non thérapeutique selon la revendication 1, dans lequel ledit mélange de régénération capillaire comprend en outre un polymère d'amidon modifié de manière cationique.

9. Procédé non thérapeutique selon la revendication 1, dans lequel ledit shampooing comprend un tensioactif détersif et un exfoliant chimique.

10. Procédé non thérapeutique selon la revendication 1, dans lequel ladite composition de shampooing comprend un agent de traitement huileux.
